# EUROPÄISCHE PATENTANMELDUNG

(11) **EP 1 319 392 A2**
(43) Veröffentlichungstag der Anmeldung: **18.06.2003**
(21) Anmeldenummer: 02026708.4
(22) Anmeldetag: 30.11.2002
(51) Int. Cl.: A61K 7/13

(54) **Melaninbildendes Haarfärbungsprodukt**

(30) Priorität: 12.12.2001 DE 10160966
(71) Anmelder: Beiersdorf AG, 20245 Hamburg (DE)
(72) Erfinder: Berens, Werner, 22307 Hamburg (DE); Smuda, Christoph, Dr., 25474 Bönningstedt (DE); Wolber, Rainer, Dr., 22399 Hamburg (DE); Stäb, Franz, Dr., 21379 Echem (DE); Blatt, Thomas, Dr., 22880 Wedel (DE); Giesen, Kyra, Dr., 22081 Hamburg (DE)

(57) **Zusammenfassung**

Haarfärbemittel enthaltend mindestens eine Verbindung aus der Gruppe der AGEs (Advanced Glycosylation Endproducts), deren synthetischen Vorstufen und/oder der Lipofuscine.

## Beschreibung

Die vorliegende Erfindung betrifft Haarfärbemittel enthaltend mindestens eine Verbindung aus der Gruppe der AGEs (Advanced Glycosylation Endproducts), deren synthetischen Vorstufen und/oder der Lipofuscine.

Der ganze menschliche Körper ist, mit Ausnahme der Lippen, der Handinnenflächen und der Fußsohlen, behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es das Erscheinungsbild des Menschen wesentlich prägt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar in den Wachstumsphasen (Anagen) neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar kann unterschiedliche Farben aufweisen. Verantwortlich für die persönliche Haarfarbe ist Melanin das als Farbstoff in den Haaren eingelagert ist. Gebildet wird Melanin in den Melanozyten. Dies sind Zellen, die in der Haarzwiebel assoziiert mit den Keratinozyten des Haarmarks vorkommen. Melanozyten enthalten als charakteristische Zellorganellen Melanosomen, in denen das Melanin gebildet wird. Dieses wird über die langen Dendriten der Melanozyten in die Keratinozyten der präkortikalen Matrix transferiert und ruft die mehr oder weniger ausgeprägte blonde bis braun-schwarze Haarfarbe hervor.

Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. DHICAund DHI-Melanin entstehen über die gemeinsamen Zwischenstufen Dopachinon und Dopachrom. Letzteres wird entweder zu Indol-5,6-Chinon-Carbonsäure oder zu lndol-5,6-Chinon umgesetzt, aus denen die beiden genannten Eumelanine entstehen.

Die Entstehung von Phäomelanin läuft über die Zwischenprodukte Dopachinon und Cysteinyldopa.

Das Eumelanin ist ein Schwarz-Braun-Pigment. Es entscheidend hauptsächlich über die Farbtiefe des Haares. In braunem und schwarzem Haar kommt es in deutlich erkennbaren Körnchen vor.

Das Phaeomelanin ist ein Rot-Pigment. Es ist verantwortlich für hellblonde, blonde und rote Haare. Phaeomelanin ist von seiner Struktur her sehr viel feiner und kleiner als Eumelanin.

Aus den verschiedenen Anteilen der Melanintypen entstehen die verschiedenen Haarfarben:
- Blondes Haar enthält wenig Eumelanin und viel Phaeomelanin.
- Dunkles Haar enthält viel Eumelanin und wenig Phaeomelanin.
- Rotes Haar hat ebenfalls wenig Eumelanin und sehr viel Phaeomelanin.

Alle dazwischenliegenden Haarschattierungen entstehen aus unterschiedlichen Mischungsverhältnissen der beiden Melanintypen.

Die Produktion der für die Haarpigmentbildung benötigten Tyrosine nimmt mit zunehmenden Lebensalter ab. Das fehlende Melanin wird durch die Einlagerung von Luftbläschen ersetzt. Die Haare erscheinen grau.

Dieser Prozeß ist in der Regel schleichend. Er beginnt an den Schläfen und weitet sich dann auf die gesamte Kopfbehaarung aus. Danach erwischt es den Bart und die Augenbrauen. Zuletzt sind schließlich alle Haare des Körpers grau.

Medizinisch werden graue Haare als Canities bezeichnet. Es gibt verschiedene Möglichkeiten des Ergrauens. Vorzeitiges Ergrauen, ab dem 20 Lebensjahr, nennt sich auch Canities praecox.

Die Canities symptomatica, oder symptomatisches Ergrauen der Haare, kann verschiedene Ursachen haben. Dazu gehören:
- Perniziöse Anämie (Vitamin-B-Mangelanämie),
- schwere endokrinologische Störungen, z. B. bei Schilddrüsenerkrankungen.
- akute, fieberhafte Erkrankungen,
- Arzneimittelnebenwirkungen,
- Kosmetika,
- Metalle.

Graue Haare werden von vielen Menschen als unattraktiv empfunden und mit Alter und körperlichen Verfall gleichgesetzt. Um das als Makel empfundene Ergrauen der Haare zu kaschieren, pflegen sich viele Menschen ihre Haare zu färben.

Schon in der Antike griffen die Menschen zu Haarfärbemitteln. Doch erst mit der Entwicklung synthetischer Farbstoffe gegen Ende des 19. Jahrhunderts erlebte die Anwendung von Haarfärbemitteln ihren Durchbruch. Man schätzt, dass heutzutage in den Industrieländern etwa 40 % aller Frauen sich die Haare färbt und auch bei Männern erfreuen sich Haarfärbemittel wachsender Beliebtheit.

Den Verbrauchern stehen eine Vielzahl von Anwendungsformen für die dauerhafte oder vorübergehende Verfärbung des Haares zur Verfügung. Neben Blondierungsmitteln, die das Haar entfärben gibt es auch eine Vielzahl von farbgebenden Mitteln.

Haarfärbemittel können in der Regel in drei Klassen eingeteilt werden:
■ direktziehende temporäre
■ semipermanente
■ permanente Färbemittel

Mit einem Marktanteil von etwa 80 % sind die permanenten Haarfärbemittel am weitesten verbreitet. Bei diesem Färbesystem werden die Farbstoffe durch chemische Reaktionen direkt auf und im Haar gebildet. Es laufen dabei Oxidationsreaktionen, Kupplungsvorgänge bzw. Kondensationsreaktionen ab, die durch Wasserstoffperoxid, in Gegenwart von Ammoniak oder Monoethanolamin hervorgerufen werden. Die Verwendung von Wasserstoffperoxid als Oxidationsmittel ist deshalb erforderlich, weil es nicht nur die Farbstoffbildung initiiert, sondern gleichzeitig auch die Melanin-Pigmente, die dem Haar seine Färbung geben, zerstört und auf diese Weise eine Blondierung hervorruft, weshalb man bei diesem Färbevorgang auch von einer aufhellenden Färbung spricht (W. Umbach (Hrsg.): Kosmetik; Entwicklung, Herstellung und Anwendung kosmetischer Mittel, 2. Aufl. Georg Thieme-Verlag, Stuttgart, 1995).

Der Einsatz von Wasserstoffperoxid, Ammoniak und anderen Chemikalien, belastet das Haar und zerstört seine Oberfläche. Das natürlich gesunde Aussehen und der seidige Glanz des Haares gehen beim Färben regelmäßig verloren. Durch die Zerstörung des Melanins im Haar durch Wasserstoffperoxid wird darüber hinaus der natürliche Schutz von Haar und Kopfhaut vor der schädlichen UV-Strahlung des Sonnenlichtes geschädigt.

Es war daher die Aufgabe der vorliegenden Erfindung die Mängel des Standes der Technik zu beseitigen Haarfärbemittel zu entwickeln mit denen sich das Haar auf schonende Weise färben läßt.

Überraschend gelöst wird die Aufgabe durch Haarfärbemittel enthaltend mindestens eine Verbindung aus der Gruppe der AGEs (Advanced Glycosylation Endproducts), deren synthetischen Vorstufen und/oder der Lipofuscine. Mit Hilfe dieser Zubereitungen kann die Melaninproduktion angeregt, und vermehrt der natürliche Farbstoff Melanin in das Haar eingelagert werden. Mit dieser überaus schonenden Art der Färbung wird die Haarfarbe intensiviert und das Haar dunkel eingefärbt. Da die Haaroberfläche nicht von aggressiven Chemikalien angegriffen wird, behält das Haar seine natürliche Schönheit und seinen seidigen Glanz.

Es ist dabei erfindungsgemäß von Vorteil, wenn die Verbindungen aus der Gruppe der AGEs, deren synthetischen Vorstufen und/oder der Lipofuscine in den erfindungsgemäßen Zubereitungen in einer Konzentration von 0,001 bis 15 Gewichts-%, insbesondere in einer Konzentration von 0,01 bis 5 Gewichts-% und ganz besonders bevorzugt in einer Konzentration von 0,1 bis 2 Gewichts-% bezogen auf das Gesamtgewicht der Zubereitung enthalten sind.

Die Bezeichnung AGE kommt vom englischen Begriff "Advanced Glycosylation Endproducts". AGEs im Sinne der vorliegenden Erfindung sind beispielsweise erhältlich gemäß der folgenden (beispielhaften) Reaktionsgleichung:

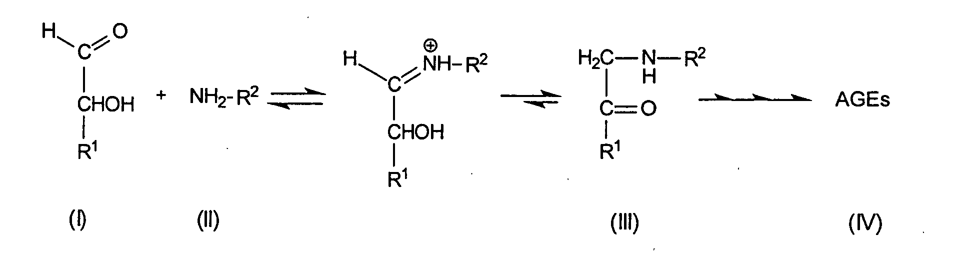

Vorteilhaft im Sinne der vorliegenden Erfindung sind neben den eigentlichen AGEs auch deren Vorläufer, wie z. B. Amadori- oder Maillard-Produkte (entsprechend (III) in obiger Reaktionsgleichung) bzw. sogenannte EGEs (Early Glycosylation Endproducts), welche beispielsweise als Zwischenprodukte bei der Umlagerung von den Amadoriprodukten (III) zu den AGEs (IV) erhältlich sind.

Vorteilhafte Ausgangsstoffe (I) tragen eine Aldehydgruppe (CHO) und werden beispielsweise aus der Gruppe der Zucker (Aldosen, z. B. Triosen, Tetrosen, Pentosen, Hexosen und dergleichen wie Glucose, Galactose, Mannose usw.) gewählt.

Vorteilhafte Ausgangsstoffe (II) weisen eine freie Aminogruppe auf und werden beispielsweise aus der Gruppe der Aminosäuren und der Gruppe der Peptide mit endständigen Aminogruppen gewählt, insbesondere Lysin, Hydroxylysin, Arginin, Tryptophan und Histidin sind vorteilhaft. Ferner vorteilhaft kann die freie Aminogruppe auch aus N-terminalen Enden von Proteinen und Peptiden stammen, ferner auch vorteilhaft Sphingosin sowie Dihydrosphingosin und deren Homologen mit verschieden langen (ungesättigten) Acylresten.

Bevorzugt enthalten erfindungsgemäße AGEs mindestens einen stickstoffhaltigen Fünfund/oder Sechsring.

Vorteilhaft im Sinne der vorliegenden Erfindung ist beispielsweise das sogenannte FFI, welches sich durch die folgende Strukturformel auszeichnet:

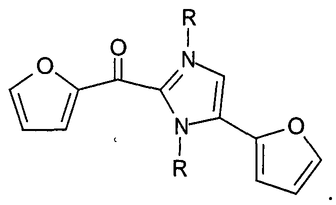

Ferner vorteilhaft im Sinne der vorliegenden Erfindung ist das sogenannte AFGP, welches sich durch die folgende Strukturformel auszeichnet:

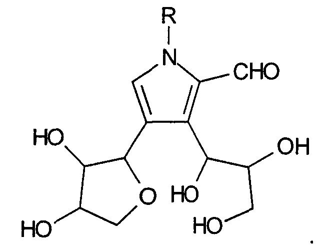

Ferner vorteilhaft im Sinne der vorliegenden Erfindung sind Pyrraline, welche sich durch die folgende Strukturformel auszeichnen:

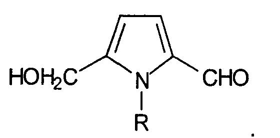

Erfindungsgemäß vorteilhaft stammt der Rest R beispielsweise aus Proteinen und Peptiden; dementsprechend handelt es sich hierbei vorzugsweise um Peptid-, Aminosäureoder Proteinreste, welche terminal oder seitlich an das Stickstoffatom angebunden sein können.

R wird ferner vorteilhaft beispielsweise aus der Gruppe der gesättigten oder ungesättigten, verzweigten oder unverzweigten Alkylgruppen mit 1 bis 35 Kohlenstoffatomen gewählt.

Ferner vorteilhaft im Sinne der vorliegenden Erfindung ist Pentosidin, welches sich durch die folgende Strukturformel auszeichnet:

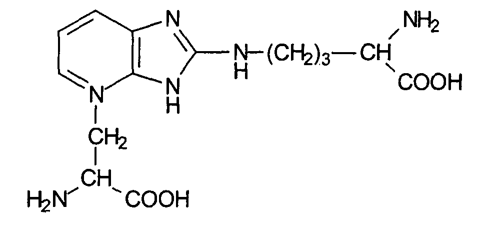

Besonders vorteilhaft im Sinne der vorliegenden Erfindung ist ferner das sogenannte A2E, welches sich durch die folgende Strukturformel auszeichnet:

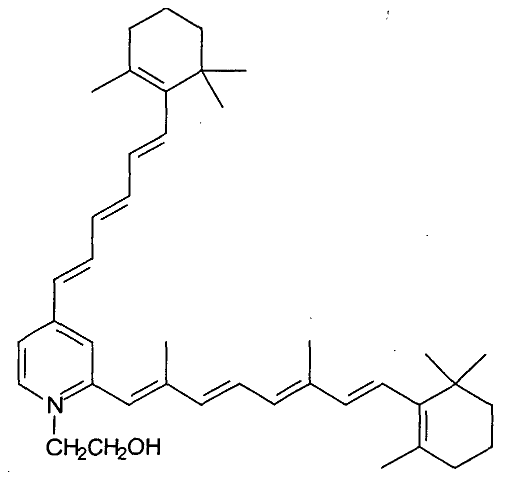

Die dargestellten Strukturformeln sollen die Erfindung selbstverständlich nicht auf bestimmte Isomeren der erfindungsgemäßen Substanzen beschränken. Vielmehr sind auch nicht dargestellte Isomere bzw. Isomerengemische vorteilhaft im Sinne der vorliegenden Erfindung.

Lipofuscine im Sinne der vorliegenden Erfindung entstehen im Rahmen natürlicher Prozesse innerhalb der menschlichen Zellen. Lipofuscin ist ein heterogenes, kohlenhydrat-, protein- und cholesterin- bzw. lipidhaltiges, gelbliches bis braunes Pigment, das in Form von Granula im Plasma verschiedener Körperzellen vorkommt. Lipofuscine treten v. a. im Alter als sogenanntes Abnutzungspigment z. B. in Nerven- und Herzmuskelzellen auf. Lipofuscin-Granula werden wahrscheinlich aus Lysosomen gebildet, in denen sich nicht weiter verwertbare Zellbestandteile angesammelt haben. Im Alter reichern sie sich im Cytoplasma somit auch in der Haut an.

Erfindungsgemäße Lipofuscine sind im Rahmen von lipidperoxidativen Prozessen erhältlich; die mehrstufigen Reaktionen laufen unter anderem über Malonaldehyde, die mit primären Aminen über Schiff sche Base-Reaktionen zur Bildung von Lipofuscinen führen.

Vorteilhafte Ausgangsstoffe für derartige Reaktionen sind Aldehyde, welche aus lipidperoxidativen Prozessen stammen, wie beispielsweise Malonaldehyde und Malodialdehyde. Diese reagieren bevorzugt mit Substanzen, welche Aminogruppen enthalten, wobei letztere vorteilhaft ebenfalls aus Lipidkomponenten stammen. Ferner vorteilhaft kann die freie Aminogruppe auch aus N-terminalen Enden von (Lipo-) Proteinen und Peptiden stammen. Besonders vorteilhafter Ausgangsstoff sind Phosphatidylethanolamin, Sphingosin sowie Dihydrosphingosin und deren Homologen mit verschieden langen (ungesättigten) Acylresten.

Bevorzugt enthalten erfindungsgemäße Lipofuscine mindestens eine stickstoffhaltige Ringstruktur.

Erfindungsgemäße Lipofuscine sind ferner z. B. erhältlich durch artifizielle chemische (z. B. mit Wasserstoffperoxid) oder physikalische (durch Einwirkung von UV-A-Strahlung) Oxidation von Liposomen - insbesondere von Liposomen, welche Phosphatidylethanolamin enthalten - und/oder durch Oxidation von isolierten, zellulären Organellen (Mitochondrien) und/oder Membranen.

Ferner vorteilhaft im Sinne der vorliegenden Erfindung sind Lipofuscine, welche sich durch die folgende Strukturformel auszeichnen: 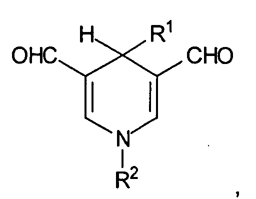 worin R¹ und R² - je nach Wahl der Ausgangsstoffe - unabhängig voneinander unterschiedlichste Reste darstellen können, vorteilhaft aber Peptid-, Aminosäure- oder Proteinreste. Es kann sich gleichermaßen um Phospholipide (Phosphatidylethanolamin) oder Sphingosine oder Dihydrosphingosine bzw. Bestandteile daraus handeln.

Weiterhin vorteilhaft im Sinne der vorliegenden Erfindung sind Lipofuscine, welche sich als lineare Kondensationsprodukte durch Schiff'sche Base-Reaktionen aus primären Aminen und Malondialdehyd (R¹-N=CH-CH=CH-N-R²) oder Hydroxyketosen ableiten, worin R¹ und R² - je nach Wahl der Ausgangsstoffe - unabhängig voneinander unter-schiedlichste Reste darstellen können, vorteilhaft aber Peptid-, Aminosäure- oder Proteinreste. Es kann sich gleichermaßen um Phospholipide (Phosphatidylethanolamin) oder Sphingosine oder Dihydrosphingosine bzw. Bestandteile daraus handeln.

Erfindungsgemäß besonders bevorzugte Lipofuscine werden aus der Gruppe 1-Amino-3-Iminopropen und dessen N,N'-substituierten Derivaten sowie 1,4-Dihydropyridin-3,5-Dicarbaldehyd und dessen N,N'-substituierten Derivaten gewählt.

Die erfindungsgemäßen Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Verdicker, Antischuppenwirkstoffe, Komplexbildner, Vitamine, Pflanzenextrakte,, Haarglättungsmittel (Entkräuselungsmittel), Farbstoffe, Pigmente, die eine färbende Wirkung haben, Verdickungsmittel, anfeuchtende und/oder feuchthaltende Substanzen, Emulgatoren, Fette, Öle, Wachse oder andere übliche Bestandteile einer kosmetischen oder dermatologischen Formulierung wie Alkohole, Polyole, Polymere, Schaumstabilisatoren, Elektrolyte, quartäre Ammoniumverbindungen, organische Lösungsmittel und/oder Silikonderivate sowie Moisturizer. Auch können die erfindungsgemäßen Zubereitungen mit den aus der Haarpflege bekannten Filmbildnern, Seifen und Tensiden in erfindungsgemäß vorteilhafter Weise kombiniert werden.

Als Filmbildner können in erfindungsgemäß vorteilhafter Weise Mischpolymerisate aus Vinylpyrrolidon und Vinylacetat oder neutralisierter Crotonsäure eingesetzt werden. Auch Terpolymere, anionische, kationische, amphotere und nichtionische Polymere können erfindungsgemäß vorteilhaft eingesetzt werden. Erfindungsgemäß vorteilhafte Filmbildner sind beispielsweise Terpolymere aus Vinylpyrrolidon, Dimethylaminopropylmethacrylamid und quarternisiertem Dimethylaminopropylmethacrylamid.

Erfindungsgemäß vorteilhafte anionische Polymere können aus der Gruppe Vinylacetat/Crotonsäure-Copolymer, Vinylacetat/Acrylat-Copolymer, Vinylacetat/Vinylneodecanoat/Crotonsäure-Copolymer, Natriumacrylat/Vinylalkohol-Copolymer, Natriumpolystyrolsulfat, Ethylacrylat/N-tert.-Butylacrylamid/Acrylsäure-Copolymer, VinylpyrrolidonNinylacetat/ltaconsäure-Copolymer, Acrylsäure/Acrylamid-Copolymer und deren Natriumsalze, sowie Homo-und/oder Copolymere von Acrylsäure und/oder Methacrylsäure und/oder deren Salze, Acrylat/Hydroxyacrylat-Copolymer, Octylacrylamid/Acrylat-Copolymer, Octylacrylamid/Methacrylsäureester-Copolymer, Butylacrylat/N-Vinylpyrrolidon-Copolymer, Methylvinylether/Maleinsäure-Copolymer und deren Ethyl-, Isopropyl- und Butylester, Silikon/Acrylsäure oder Methacrylsäure Copolymer, Diglcol/Cyclohexandimethanol/lsophthalat/Sulfoisophthalat Copolymer, Polyurethane auf Basis von Diisocyanaten mit endständigen Säuregruppen gewählt werden.

Es ist ferner erfindungsgemäß vorteilhaft, das amphotere Polymer aus der Gruppe N-Octylacrylamid/Acrylsäure/tert.-Butylaminoethylmethacrylat-Copolymer, Gruppe N-Octylacrylamid/Methacrylsäure/tert.-Butylaminoethylmethacrylat-Copolymer sowie Copolymere aus Methacryloylbetain / Alkylmethacrylaten, Copolymere aus Monomeren mit Carboxy- und/oder Sulfongruppen, insbesondere Acrylsäure, Methacrylsäure, Itaconsäure und Monomeren mit Aminogruppen, insbesondere Monoalkylaminoalkylacrylate, Dialkylaminoalkylacrylate, Monoalkylaminoalkylmethacrylate, Dialkylaminoalkylmethacrylate, Monoalkylaminoalkylacrylamide, Dialkylaminoalkylacrylamide, Monoalkylaminoalkylmethacrylamide, Dialkylaminoalkylmethacrylamide, und Copolymeren aus N-Octylacrylamid, Methylmethacrylat, Hydroxypropylmethacrylat, N-tert.-Butylaminoethylmethacrylat und Acrylsäure zu wählen.

Erfindungsgemäß vorteilhafte kationische Polymere sind beispielsweise Polyquarterrnium-4 (Hydroxyethylcellulosedimethyldiallylammoniumchlorid-Copolymer), Polyquarternium-11 (quarternisiertes Vinylpyrrolidonalkylaminomethacrylat) oder Polyquarternium-16, (Methylvinylimidazoliumvinylpyrolidon), Cetyltrimethylammoniumchlorid und Cetyldimethylammoniumphosphat.

Erfindungsgemäß vorteilhafte, nichtionische Polymere können beispielsweise aus der Gruppe der folgenden Verbindungen gewählt werden:
- Homopolymere des N-Vinylpyrrolidons
- Homopolymere des N-Vinylcaprolactams
- Homopolymere des N-Vinylformamids
- Copolymere aus N-Vinylpyrrolidon und Vinylacetat und/oder Vinylpropionat
- Terpolymere aus N-Vinylpyrrolidon, Vinylacetat und Vinylpropionat
- Terpolymere aus N-Vinylpyrrolidon, Acrylamid und Vinylalkohol.

Als Tenside können anionische, kationische, amphotere und nichtionische Tenside, wie sie beispielsweise in Haarshampoos Verwendung finden, erfindungsgemäß vorteilhaft eingesetzt werden.

Anionische Tenside weisen als funktionelle Gruppen in der Regel Carboxylat-, Sulfatoder Sulfonatgruppen auf. In wässriger Lösung bilden sie im sauren oder neutralen Milieu negativ geladene organische lonen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische lonen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wässrigem Medium keine lonen.

Besonders vorteilhafte waschaktive anionische Tenside im Sinne der vorliegenden Erfindung sind
Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,
Sulfonsäuren und deren Salze, wie
■ Acyl-isethionate, z.B. Natrium-/ Ammoniumcocoyl-isethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
   sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Besonders vorteilhafte waschaktive kationische Tenside im Sinne der vorliegenden Erfindung sind quarternäre Tenside. Quaternäre Tenside enthalten mindestens ein N-Atom, das mit 4 Alkyl- oder Arylgruppen kovalent verbunden ist. Vorteilhaft sind Benzalkoniumchlorid, Alkylbetain, Alkylamidopropylbetain und Alkyl-amidopropylhydroxysultain.

Besonders vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Besonders vorteilhafte waschaktive nicht-ionische Tenside im Sinne der vorliegenden Erfindung sind
■ Alkanolamide, wie Cocamide MEA/ DEA/ MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat,
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole und
■ ethoxylierte Amine.

Weitere geeignete nicht-ionische Tenside im Sinne der vorliegenden Erfindung sind ferner Aminoxide, wie Cocoamidopropylaminoxid.

Vorteilhaft liegen Zubereitungen gemäß der Erfindung in Form von Gelen vor und enthalten einen oder mehrere Gelbildner bzw. Hydrokolloide.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wäßrigen Medien ist. Voraussetzung dafür ist, daß diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

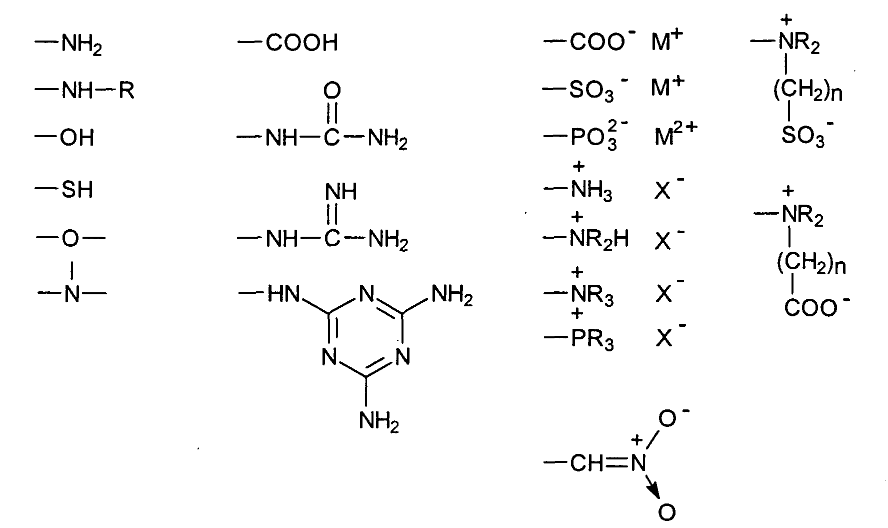

Die Gruppe der kosmetisch und dermatologisch relevanten Hydrokolloide läßt sich wie folgt einteilen in:
organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
organische, abgewandelte Naturstoffe, wie z. B. Carboxymethylcellulose und andere Celluloseether, Hydroxyethyl- und -propylcellulose und dergleichen,
organische, vollsynthetische Verbindungen, wie z. B. Polyacryl- und Polymethacryl-Verbindungen, Vinylpolymere, Polycarbonsäuren, Polyether, Polyimine, Polyamide,
anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus 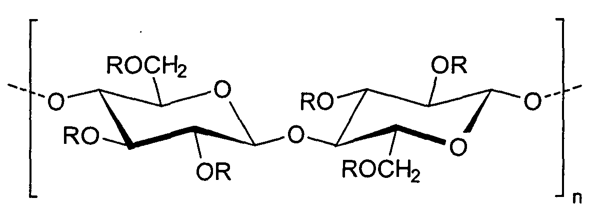 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH₂-COONa darstellen kann. Bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2×10⁶ bis 24×10⁶ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 10⁶ produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlant., zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht lösl. ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-Lgalactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute τ-Carrageenan ist sowohl wasserlöslich als auch gelbildend. Weitere Carrageen-Typen werden ebenfalls mit griechischen Buchstaben bezeichnet. Auch die Art vorhandener Kationen (K⁺, NH₄⁺, Na⁺, Mg²⁺, Ca²⁺) beeinflußt die Löslichkeit der Carrageene.

Polyacrylate sind ebenfalls vorteilhaft im Sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

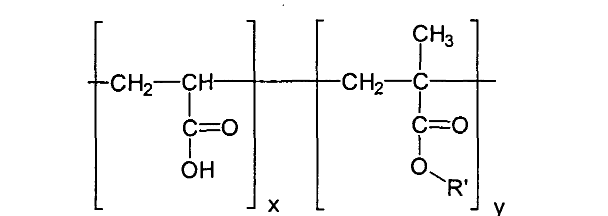

Darin stellen R' einen langkettigen Alkylrest und x und y Zahlen dar, welche den jeweiligen stöchiometrischen Anteil der jeweiligen Comonomere symbolisieren.

Erfindungsgemäß bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C₁₀₋₃₀ -Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C ₁₀₋₃₀ Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCI-Bezeichnung "acrylates/C₁₂₋₂₄ pareth-25 acrylate copolymer" (unter der Handelsbezeichnungen Synthalen® W2000 bei der 3V Inc. erhältlich), die die INCI-Bezeichnung "acrylates/steareth-20 methacrylate copolymer" (unter der Handelsbezeichnungen Aculyn® 22 bei der International Specialty Products Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C₁₀₋₃₀ alkyl PEG-20 itaconate copolymer" (unter der Handelsbezeichnungen Structure Plus® bei der National Starch erhältlich) und ähnliche Polymere.

Die jeweils einzusetzenden Mengen an kosmetischen oder dermatologischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Ferner ist es vorteilhaft, wenn die Zubereitungen im Sinne der vorliegenden Erfindung Enzyme bzw. Enzym-Mimics enthalten, die als enzymatische Antioxidantien wirken, wie z. B. Superoxiddismutasen, Katalasen, Peroxidasen, Selenoproteine.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist auch von Vorteil, den erfindungsgemäßen Zubereitungen Komplexbildner zuzusetzen. Vorteilhaft werden die Komplexbildner gewählt aus der Gruppe bestehend aus Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen, Tetrasodium Iminodisuccinate, Trisodium Etylenediamine Disuccinate.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Formulierungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi und/oder Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Vorzugsweise enthalten die erfindungsgemäßen Zubereitungen neben den erfindungsgemäßen Wirkstoffen zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten. Als UV-Filtersubstanzen können alle bekannten und für die Kosmetik zugelassenen UV-Filtersubstanzen eingesetzt werden.

Die erfindungsgemäßen kosmetischen und/oder dermatologischen Zubereitungen für das Haar können vorteilhaft als Emulsion, Lotion, Gel, wässrige Lösung oder Öl in Form eines Shampoos, einer Creme, eines Haarwassers, eines Sprays, einer Pomade, einer Spülung, eines Pulvers oder einer Kurpackung vorliegen.

In den Zubereitungen können im Sinne der vorliegenden Erfindung neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Emulsionen können vorzugsweise auch eine Mikroemulsion, eine Pickering-Emulsion oder eine sprühbare Emulsion sein. Darüber hinaus können die erfindungsgemäßen Formulierungen aber auch vorteilhaft in Form von ölfreien Zubereitungen oder als wasserfreie Zubereitungen vorliegen.

Zur Anwendung werden die erfindungsgemäßen kosmetischen und dermatologischen Zubereitungen in der für Kosmetika üblichen Weise auf die die Haare in ausreichender Menge aufgebracht.

Erfindungsgemäß ist auch das Verfahren zur Färbung der Haare, welches dadurch gekennzeichnet ist, dass die Haare und/oder die Kopfhaut mit einem Haarfärbemittel enthaltend mindestens eine Verbindung aus der Gruppe der AGEs, deren synthetischen Vorstufen und/oder der Lipofuscine behandelt wird.

Erfindungsgemäß ist auch die Verwendung der erfindungsgemäßen Zubereitungen zur Steigerung der Melaninsynthese in der Haarwurzel und der Einlagerung von Melanin im Haar.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Herstellungsbeispiel für Lipofuscin

Leber-Gewebe wird in 0,3M Sucrose auf Eis homogenisiert und anschließend für 10 Minuten bei 270 g (1g steht hier und im nachfolgenden für die einfache Erdbeschleunigung) zentrifugiert. Der Überstand hieraus wird ein weiteres mal bei 1100 g über 20 Minuten zentrifugiert, das entstehende Sediment verworfen und der Überstand abermals zentrifugiert (diesesmal 10 Minuten bei 3000 g). Das aus der letzten Zentrifugation verbleibende Sediment, das überwiegend aus Mitochondrien besteht, wird ins PBS (Phosphate Bufferd Saline, ein einfacher Phosphat-Puffer bei pH 7,4) aufgenommen und wie zuvor zentrifugiert (Waschschritt). Das verbleibende, von Sucrose weitgehend gereinigte Pellet wird in 2ml PBS aufgenommen und unter der sterilen Werkbank 20 Stunden mit UV-Licht bestrahlt. Während dieser Zeit findet die Lipidperoxidation (u.a. Entstehung von Malondialdehyd) statt, was über Schiff'sche Basen-Reaktionen mit freien Aminogruppen (aus Proteinen und Phosphatidylethanolamin der Mitochondrien-Membranen) zur Bildung von Lipofuscin führt.

### Herstellung von A2E

Die Herstellung von A2E kann nach der Methode von Parish (Parish et *al.,* 1998, Proc. Natl. Acad. Sci. USA, 95:14609-13) erfolgen.

Das so gewonnene Lipofuscin bzw. A2E wurde in den nachfolgenden Rezepturbeispielen eingesetzt.

### Beispiele

| **Sprayformulierung** | **1** |
|---|---|
| Ethanol | 28,00 |
| Lipofuscin | 0,3 |
| Ubiquinon Q10 | 0,10 |
| Konservierungsmittel, Farbstoffe, Parfüm | q.s. |
| Propan/Butan 25/75 | ad 100 |

| **Duschbad** | **2** |
|---|---|
| Natriumlaurethsulfat | 33,00 |
| Kaliumcocoyl hydrolysiertes Collagen (30%) | 11,00 |
| Cocoamphodiacetat (30%) | 5,00 |
| PEG-7-Glyceryl Cocoate | 2,00 |
| Cocamide MEA | 1,00 |
| Natriumchlorid | 0,50 |
| A2E | 0,05 |
| Zitronensäure | 0,02 |
| Konservierungsmittel, Farbstoffe, Parfüm | q.s. |
| Wasser | ad 100 |

| **Conditioner-Shampoo mit Perlglanz** | **3** |
|---|---|
| Polyquaternium-10 | 0,50 |
| Natriumlaurethsulfat | 9,00 |
| Cocoamidopropylbetain | 2,50 |
| Perlglanzmittel | 2,00 |
| A2E | 0,1 |
| Dinatrium EDTA | 0,10 |
| Konservierungsmittel, Parfüm, Verdicker, pH-Einstellung und Lösungsvermittler | q.s. |
| Wasser, VES (voll Entsalzt) | ad 100 |
| Der pH-Wert wird auf 6 eingestellt. | |

| **klares Conditioner-Shampoo** | **4** |
|---|---|
| Polyquaternium-10 | 0,50 |
| Natriumlaurethsulfat | 9,00 |
| Cocoamidopropylbetain | 2,50 |
| A2E | 0,1 |
| Iminodibernsteinsäure, Na-Salz | 0,20 |
| Konservierungsmittel, Parfüm, Verdicker, pH-Einstellung und Lösungsvermittler | q.s. |
| Wasser, VES (voll Entsalzt) | ad 100 |
| Der pH-Wert wird auf 6 eingestellt. | |

| **klares Light-Shampoo mit Volumeneffekt** | **5** |
|---|---|
| Natriumlaurethsulfat | 9,00 |
| Cocoamidopropylbetain | 2,50 |
| A2E | 0,05 |
| Dinatrium EDTA | 0,20 |
| Konservierungsmittel, Parfüm, Verdicker, pH-Einstellung und Lösungsvermittler | q.s. |
| Wasser | ad 100 |
| Der pH-Wert wird auf 5,5 eingestellt. | |

| **Haarkur** | **6** |
|---|---|
| Hydroxypropylmethylcellulose | 0,50 |
| Cetrimoniumbromid | 1,00 |
| Glycerin | 3,00 |
| Cetearylalkohol | 2,50 |
| Glycerylstearat | 2,00 |
| A2E | 0,1 |
| Konservierungsmittel, Parfüm, pH-Einstellung | q.s. |
| Wasser | ad 100 |
| Der pH-Wert wird auf 3,5 eingestellt. | |

| **Haarspülung** | **7** |
|---|---|
| Behentrimoniumchlorid | 1,00 |
| Glycerin | 3,00 |
| Hydroxyethylcellulose | 0,20 |
| Cetearylalkohol | 3,00 |
| A2E | 0,2 |
| Folsäure | 0,80 |
| Konservierungsmittel, Parfüm, pH-Einstellung | q.s. |
| Wasser | ad 100 |
| Der pH-Wert wird auf 3,0 eingestellt. | |

## Patentansprüche

1. Haarfärbemittel enthaltend mindestens eine Verbindung aus der Gruppe der AGEs (Advanced Glycosylation Endproducts), deren synthetischen Vorstufen und/oder der Lipofuscine.

2. Haarfärbemittel nach Anspruch 1, **dadurch gekennzeichnet, dass** sie Verbindungen aus der Gruppe der AGEs, deren synthetischen Vorstufen und/oder der Lipofuscine in einer Konzentration von 0,001 bis 15 Gewichts-% bezogen auf das Gesamtgewicht der Zubereitung enthält.

3. Haarfärbemittel nach einem der Ansprüche 1 oder 2, **dadurch gekennzeichnet, dass** das oder die AGEs und/oder deren synthetischen Vorstufen aus der Gruppe der Verbindungen FFI, AFGP, Pyrralinen, Pentosidin und A2E gewählt werden.

4. Haarfärbemittel nach einem der Ansprüche 1 bis 3, **dadurch gekennzeichnet, dass** der oder die Lipofuscine aus der Gruppe 1-Amino-3-Iminopropen und dessen N,N'substituierten Derivaten sowie 1,4-Dihydropyridin-3,5-Dicarbaldehyd und dessen N,N'-substituierten Derivaten gewählt werden.

5. Haarfärbemittel nach einem der Ansprüche 1 bis 4, **dadurch gekennzeichnet**, das sie als Emulsion, Lotion, Gel, wässrige Lösung oder Öl in Form eines Shampoos, einer Creme, eines Haarwassers, eines Sprays, einer Pomade, einer Spülung, eines Pulvers oder einer Kurpackung vorliegen.

6. Verfahren zur Färbung der Haare, **dadurch gekennzeichnet, dass** die Haare und/oder die Kopfhaut mit einem Haarfärbemittel enthaltend mindestens eine Verbindung aus der Gruppe der AGEs, deren synthetischen Vorstufen und/oder der Lipofuscine nach einem der Ansprüche 1 bis 5 behandelt werden.

7. Verwendung von Haarfärbemitteln nach einem der vorhergehenden Ansprüche zur Steigerung der Melaninsynthese in der Haarwurzel und der Einlagerung von Melanin im Haar.
